Europäisches Patentamt

European Patent Office

Office européen des brevets

(11) Veröffentlichungsnummer: **0 038 446 B1**

(12) **EUROPÄISCHE PATENTSCHRIFT**

(45) Veröffentlichungstag der Patentschrift:
18.05.83

(51) Int. Cl.³: **B 01 J 23/50**, B 01 J 23/66,
C 07 D 301/10

(21) Anmeldenummer: **81102446.2**

(22) Anmeldetag: **01.04.81**

(54) **Silberhaltige Trägerkatalysatoren und Katalysator-Zwischenprodukte, Verfahren zu ihrer Herstellung und ihre Verwendung.**

(30) Priorität: **12.04.80 DE 3014091**

(43) Veröffentlichungstag der Anmeldung:
**28.10.81 Patentblatt 81/43**

(45) Bekanntmachung des Hinweises auf die Patenterteilung:
**18.05.83 Patentblatt 83/20**

(84) Benannte Vertragsstaaten:
**BE DE FR IT NL SE**

(56) Entgegenhaltungen:
**EP-A-0 002 977**
**DE-A-1 542 229**
**DE-A-2 352 608**
**DE-A-2 454 972**
**DE-A-2 461 587**

(73) Patentinhaber: **EC ERDÖLCHEMIE GMBH,
Postfach 75 2002, D-5000 Köln 71 (DE)**

(72) Erfinder: **Alter, Eduard, Dr., Narzissenweg 14,
D-6302 Lich 1 (DE)**
Erfinder: **Bruns, Ludwig, Dr., Am Kronenpützchen 26,
D-4047 Dormagen-Straberg (DE)**
Erfinder: **Volprecht, Werner, Sankt-Simeon-Strasse 8,
D-5151 Fliesteden (DE)**

(74) Vertreter: **Dill, Erwin, Dr. et al, c/o BAYER AG
Zentralbereich Patente Marken und Lizenzen
Bayerwerk, D-5090 Leverkusen 1 (DE)**

## Silberhaltige Trägerkatalysatoren und Katalysator-Zwischenprodukte, Verfahren zu ihrer Herstellung und ihre Verwendung

Die Erfindung betrifft silberhaltige und gegebenenfalls Promotoren enthaltende Trägerkatalysatoren und Katalysator-Zwischenprodukte, Verfahren zu ihrer Herstellung und ihre Verwendung zur Herstellung von Alkylenoxiden durch direkte Oxidation von Alkenen mit Luft bzw. Sauerstoff enthaltenden Gasen.

Für die industrielle Herstellung von Alkylenoxiden verwendet man heute die Direktoxidation von Alkenen mit Luft bzw. mit molekularen Sauerstoff enthaltenden Gasen in Gegenwart eines silberhaltigen Katalysators. Da Alkylenoxide, besonders Ethylenoxid, als Basischemikalien für zahlreiche Folgeprodukte eine grosse wirtschaftliche Bedeutung haben, sind viele Versuche unternommen worden, die Leistungsfähigkeit der eingesetzten Katalysatoren ständig zu verbessern. Die zahlreichen vorgeschlagenen Modifikationen zur Verbesserung der Aktivität und der Selektivität betreffen das verwendete Trägermaterial, das Verfahren zur Herstellung der Katalysatoren und den Zusatz von Promotoren (R. Landau und R. E. Lidow, «Ethylene and its industrial derivatives» Verlag S. A. Miller und Ernest Benn, London 1963; D. J. Hucknall «Selektiv oxidations of hydrocarbons» Academic Press, London 1974; J. of catalysis 34, 100–114 [1974]).

Besondere Bedeutung kommt der Arbeitstemperatur eines Katalysators für die Alkylenoxid-Herstellung zu. Wünschenswert sind Katalysatoren, die bei niedriger Temperatur eine hohe Aktivität und Selektivität aufweisen. Eine niedrige Arbeitstemperatur bewirkt beispielsweise eine längere Standzeit für den Katalysator, was für das technische Verfahren eine grosse Bedeutung hat. Weiterhin ist die Bildung von Nebenprodukten, beispielsweise bei der Ethylenoxid-Herstellung die Bildung des isomeren Acetaldehyds und des Formaldehyds, bei niedrigen Temperaturen wesentlich geringer und erleichtert somit die Abtrennung dieser Verunreinigungen bei der Aufarbeitung des Alkylenoxids zu einer reinen, allen Ansprüchen genügenden Ausgangschemikalie.

Bei hohen Arbeitstemperaturen kommt es neben den bereits geschilderten Nachteilen oftmals am Ausgang des Katalysatorbettes zu unerwünschten Nachreaktionen. Die dabei auftretenden Reaktionsprodukte können den Katalysator in seiner Leistungsfähigkeit schädigen und führen weiterhin zu unerwünschten Produktionsausfällen in kommerziell genutzten Anlagen. Hohe Arbeitstemperaturen begünstigen weiterhin das Auftreten von unkontrollierbaren Heissstellen, die neben technischen Fehlern auch eine Beeinträchtigung der Sicherheit des Herstellungsverfahrens hervorrufen können.

Die Arbeitstemperatur eines Katalysators kann man durch den Zusatz von Promotoren und durch das Herstellungsverfahren beeinflussen. Als Promotoren haben sich zugesetzte Oxide, Hydroxide und Peroxide der Alkali- und Erdalkalimetalle als besonders vorteilhaft herausgestellt (US-A-2 404 438). In einer Reihe von Patentanmeldungen, beispielsweise DE-A-2 300 512, wird vor allem der Zusatz der schweren Alkalimetalle als Promotoren beschrieben. In weiteren Patentanmeldungen, beispielsweise DE-B-1 920 976, wird unter den Erdalkalimetallen das Barium besonders als Promotor hervorgehoben.

In der DE-A-2 733 688 wird ein Verfahren zur Herstellung eines silberhaltigen Trägerkatalysators beansprucht, wobei man ein Trägermaterial mit einer Silberverbindung imprägniert, die imprägnierten Teilchen durch zumindest teilweise Umwandlung in elementares Silber aktiviert und schliesslich auf dem so hergestellten Katalysator mindestens eines der Alkalimetalle Kalium, Rubidium und Cäsium abscheidet. Die silberhaltige Imprägnierlösung enthält bevorzugt ein Bariumsalz.

Alle bisher beschriebenen Katalysatoren weisen die relativ hohe Arbeitstemperatur von 230 bis 260°C auf.

Aus der DE-OS-2 461 587 ist es bekannt, einen inerten Träger mit einer wirksamen Oberfläche von bis zu 1 m²/g mit einer Lösung von Silberlactat und Promotormetallionen wie Bariumionen in Milchsäure zu tränken und nach Trocknung mit Luftsauerstoff bei 300°C in unkontrollierter Weise zu behandeln. Mit diesem Katalysator werden Ethylenoxid-Selektivitäten von 72,5 bzw. 73% erreicht.

Aus der EP-Anmeldung 2 977 ist der Einsatz von silberhaltigen Katalysatoren für die Eox-Herstellung bekannt, bei denen Träger mit einer Oberfläche von mindestens 10 m²/g mit einer Lösung von Silberacetat in Pyridin getränkt werden. Nach einer thermischen Zersetzung von 280°C in Stickstoffatmosphäre und einer Vorbehandlung bei 168 bis 194°C in Ethylen-Luft (50:50) erhält man bei 198°C Betriebstemperatur eine Ethylenoxid-Selektivität von 71,2 bis 76%.

Es wurden nun silberhaltige und gegebenenfalls Promotoren enthaltende Trägerkatalysatoren gefunden, hergestellt durch

a) Tränken eines Trägers mit einer spezifischen Oberfläche von höchstens 1 m²/g mit einer Silber- und gegebenenfalls Promotormetall-Ionen enthaltenden Milchsäure,

b) Trocknen des nach a) erhaltenen getränkten Trägers und Vorzersetzen der in ionischer und in freier Form vorliegenden Milchsäure in praktisch sauerstofffreier Atmosphäre, wobei das Trocknen bei einer Temperatur von 50 bis 120°C vorgenommen wird und die Vorzersetzung nacheinander in den beiden Temperaturbereichen von 140 bis 220°C und 400 bis 500°C durchgeführt wird, wobei man für den Übergang zwischen beiden Temperaturbereichen eine Aufheizrate von 70 bis 150°C/Stunde einstellt, und

c) Aktivierung der nach b) erhaltenen Katalysator-Zwischenprodukte durch Erhitzen in sauerstoffhaltiger Atmosphäre, wobei die Temperatur

von mindestens 130°C auf maximal 300°C mit einer Aufheizrate von 3 bis 8°C/Stunde gesteigert wird und der Sauerstoffgehalt von 0,4 bis 21 Volumenprozent in einer solchen Weise gesteigert wird, dass im Abgas der Aktivierungsstufe ein $CO_2$-Gehalt von 1 Vol.-% nicht überschritten wird.

Die erfindungsgemässen Katalysatoren können solche mit oder ohne Promotoren sein. Für den Fall, dass die erfindungsgemässen Katalysatoren Promotoren enthalten, seien hierfür Erdalkalimetallverbindungen, beispielsweise Verbindungen von Calcium, Strontium oder Barium, und/oder Alkalimetallverbindungen, beispielsweise Verbindungen von Lithium, Natrium, Kalium, Rubidium oder Cäsium, genannt. Bevorzugte Promotoren sind Barium- und/oder Cäsium-Verbindungen. Erfindungsgemässe Katalysatoren können Verbindungen des Bariums oder des Cäsiums oder Verbindungen beider Metalle enthalten. Besonders bevorzugte erfindungsgemässe Katalysatoren sind solche, die Barium- und Cäsium-Verbindungen enthalten.

Als Mengen für die aktiven Metalle seien beispielsweise die folgenden genannt, die alle als Metalle bzw. Metallionen gerechnet sind und sich alle auf das Gesamtgewicht des fertigen Katalysators beziehen:

Für das Silber eine Menge von 7 bis 30 Gew.-%, bevorzugt 10 bis 20 Gew.-%, besonders bevorzugt 12 bis 18 Gew.-%;

falls Erdalkalimetallverbindungen als Promotoren anwesend sind, hierfür eine Menge von 0,05 bis 0,5 Gew.-%, bevorzugt 0,07 bis 0,3 Gew.-%, besonders bevorzugt 0,08 bis 0,15 Gew.-%;

für den Fall, dass Alkalimetalle als Promotoren anwesend sind, hierfür eine Menge von 0,001 bis 0,03 Gew.-%, bevorzugt 0,003 bis 0,02 Gew.-%, besonders bevorzugt 0,004 bis 0,01 Gew.-%;

falls Erdalkalimetalle und Alkalimetalle gemeinsam als Promotoren anwesend sind, können sie unabhängig voneinander in den jeweils für sie angegebenen Mengen anwesend sein.

Als Trägermaterial für die erfindungsgemässen Katalysatoren seien poröse, hitzebeständige Katalysatorträgermaterialien genannt, die sich unter den Bedingungen der Katalysator-Herstellung und der Katalysator-Verwendung inert verhalten. Das Trägermaterial hat eine makroporöse Struktur mit einer spezifischen Oberfläche von höchstens 1 m²/g, beispielsweise 0,001 bis 1 m²/g, bevorzugt 0,002 bis 0,1 m²/g, besonders bevorzugt 0,005 bis 0,05 m²/g. Die Porosität des Trägermaterials beträgt beispielsweise 40 bis 70%, bevorzugt 45 bis 60%. Für den Porendurchmesser sei beispielsweise der Bereich von 100 bis 1500 µm genannt. Als Trägermaterialien mit den genannten physikalischen Eigenschaften kommen solche natürlichen oder synthetischen Ursprungs in Betracht, beispielsweise α-Aluminiumoxid, Siliciumcarbid, synthetische oder natürliche Zeolithe, Magnesiumoxid, Zirkoniumoxid oder keramische Materialien, bevorzugt α-Aluminiumoxid.

Die Erfindung betrifft weiterhin ein Verfahren zur Herstellung von silberhaltigen und gegebenenfalls Promotoren enthaltenden Trägerkatalysatoren durch Tränken eines Trägers mit einer spezifischen Oberfläche von höchstens 1 m²/g mit einer Silber und gegebenenfalls Promotormetalle enthaltenden Milchsäure-Lösung, anschliessende Trocknung Kalzinierung, das dadurch gekennzeichnet ist, dass man

a) den getränkten Träger in einer praktisch sauerstofffreien Atmosphäre bei 50 bis 120°C trocknet und die in ionischer oder freier Form vorliegende Milchsäure nacheinander in den beiden Temperaturbereichen von 140 bis 220°C und 400 bis 500°C in praktisch sauerstofffreier Atmosphäre vorzersetzt, wobei man für den Übergang zwischen beiden Temperaturbereichen eine Aufheizrate von 70 bis 150°C pro Stunde einstellt und

b) das nach a) erhaltene Katalysator-Zwischenprodukt durch Erhitzen in einer sauerstoffhaltigen Atmosphäre aktiviert, wobei die Temperatur von mindestens 130 auf maximal 300°C mit einer Aufheizrate von 3 bis 8°/Stunde gesteigert wird und der Sauerstoffgehalt von 0,4 bis 21 Vol.-% in einer solchen Weise gesteigert wird, dass im Abgas der Aktivierungsstufe ein $CO_2$-Gehalt von 1 Vol.-% nicht überschritten wird.

Als Träger für das erfindungsgemässe Katalysator-Herstellungsverfahren sei beispielsweise einer der oben beschriebenen Träger mit den dort beschriebenen physikalischen Eigenschaften genannt. Bevorzugt wird α-Aluminiumoxid mit den oben beschriebenen Eigenschaften als Träger eingesetzt.

Als Milchsäure kann das Racemat, das die beiden optischen Antipoden in jeweils gleichen Mengen enthält, eingesetzt werden oder eine Milchsäure, die eines der optischen Antipoden im Überschuss enthält. Bevorzugt ist der Einsatz einer Milchsäure, die neben einem Gehalt an Racemat eines der optischen Antipoden im Überschuss enthält. Für diese bevorzugte Form sei beispielsweise eine Milchsäure genannt, die mindestens 50 Gew.-% eines optischen Antipoden, bevorzugt mindestens 50 Gew.-% der L(+)-Form, besonders bevorzugt mindestens 80 Gew.-% der L(+)-Form enthält, wobei der Rest jeweils aus dem Racemat der beiden optisch aktiven Formen besteht.

Die erfindungsgemäss zum Tränken des Katalysatorträgers eingesetzte Milchsäure enthält Silber in Form von Silberionen. Für die Herstellung einer solchen silberhaltigen Milchsäure sei beispielsweise das Eintragen von Silberoxid, Silbercarbonat oder separat hergestelltem Silberlactat in die Milchsäure genannt. Selbstverständlich können auch andere in der Hitze zersetzliche Silberverbindungen hierfür verwendet werden. Bevorzugt ist das Eintragen von Silberoxid, besonders bevorzugt von frisch gefälltem Silberoxid, in die Milchsäure. Die zum Tränken verwendete Milchsäure enthält beispielsweise 30 bis 45 Gew.-%, bevorzugt 35–40 Gew.-% Silberionen, bezogen auf die Gesamtmenge an Tränkflüssigkeit. Der gewünschte Gehalt an Silberionen kann gegebenenfalls vor dem Tränken durch Zugabe von destilliertem Wasser zur Tränkflüssigkeit auf das gewünschte Mass eingestellt werden. Die Herstellung der silberhaltigen Milchsäure erfolgt bei einer Temperatur von beispielsweise 40 bis 70°C. Nach Beendigung des Zusatzes der Silberverbindung wird der Milchsäure zweckmässigerweise noch eine Menge von etwa 1 ml 30 gew.-%iger Wasserstoffperoxid-Lösung/100 g Silberionen zugesetzt.

Für den Fall, dass Erdalkali- und/oder Alkalimetall-Ionen als Promotoren zugesetzt werden, sei ein Zusatz von Hydroxiden oder Carbonaten eines oder mehrerer Erdalkali- und/oder Alkalimetalle genannt. Für die Erdalkalimetalle seien beispielsweise folgende Verbindungen genannt: Calciumhydroxid, Calciumcarbonat, Strontiumhydroxid, Strontiumcarbonat, Bariumhydroxid, Bariumcarbonat, bevorzugt Bariumhydroxid oder Bariumcarbonat. Als Alkalimetallverbindungen seien beispielsweise genannt: Lithiumhydroxid, Lithiumcarbonat, Natriumhydroxid, Natriumcarbonat, Kaliumhydroxid, Kaliumcarbonat, Rubidiumhydroxid, Rubidiumcarbonat, Cäsiumhydroxid, Cäsiumcarbonat, bevorzugt Cäsiumhydroxid oder Cäsiumcarbonat. Als Menge sei beispielsweise bei den Erdalkalimetallverbindungen eine solche von 0,5 bis 3, bevorzugt 1 bis 2 g/100 g 100%iger Milchsäure genannt. Für die Alkalimetallverbindungen sei beispielsweise eine Menge von 30 bis 300, bevorzugt 70 bis 150 mg/100 g 100%iger Milchsäure genannt.

Die Menge der angesetzten, das Silber und gegebenenfalls die Promotormetalle enthaltenden Milchsäure sowie die Mengen der darin enthaltenden aktiven Metalle richten sich selbstverständlich nach der gewünschten Menge der aktiven Metalle auf dem fertigen Katalysator im Rahmen der obengenannten Bereiche für die einzelnen Metalle und weiterhin nach der Porosität des eingesetzten Katalysatorträgers. Diese Zusammenhänge können jedoch in einfachen Vorversuchen ermittelt werden. Das Volumen der zum Tränken eingesetzten Milchsäurelösung wird so bemessen, dass der Katalysatorträger vollständig getränkt wird, jedoch nach dem Tränken nur wenig Tränkungsflüssigkeit vom getränkten Katalysator abtropft. Beispielsweise sei als vom getränkten Träger abtropfende Milchsäurelösung eine Menge von 5 bis 30, bevorzugt 10 bis 20 Vol.-% der gesamten eingesetzten Milchsäurelösung genannt.

In einer bevorzugten Variante des erfindungsgemässen Verfahrens können der Silber und gegebenenfalls Promotormetalle enthaltenden Milchsäure lösliche, leicht zersetzliche, nicht reduzierende organische Verbindungen zugesetzt werden, wie Zuckeralkohole (Sorbit, Mannit), Polyhydroxysäuren, Saccharose, Stärke, Trimellithsäure, bevorzugt Saccharose. Diese genannten organischen Verbindungen werden beispielsweise in einer Menge von 30 bis 45 g/100 g 100%iger Milchsäure zugesetzt.

Die Reihenfolge der Zugabe der einzelnen Komponenten für die Tränkungsflüssigkeit kann beispielsweise die folgende sein: Vorlegen der Milchsäure, gegebenenfalls Eintragen der Erdalkalimetallverbindung, gegebenenfalls Eintragen der Alkalimetallverbindung, Eintragen der Silberverbindung, zweckmässigerweise Zusatz des Wasserstoffperoxids und gegebenenfalls Zusatz der beschriebenen organischen Verbindung. Das Eintragen der genannten Komponenten in die vorgelegte Milchsäure kann jedoch auch in beliebiger anderer Reihenfolge erfolgen.

Mit der in der beschriebenen Weise erhaltenen Tränkungsflüssigkeit wird ein oben beschriebener Katalysatorträger durch ein- oder mehrmaliges Eintauchen in die Lösung getränkt. Den beschriebenen Überschuss der Tränkungsflüssigkeit lässt man frei abtropfen und bringt sodann den getränkten Träger in einen Umluftofen.

In diesem Umluftofen wird eine praktisch sauerstofffreie Atmosphäre aufrecht erhalten. Als praktisch sauerstofffreie Atmosphäre sei eine solche genannt, die höchstens 100 Vol.-ppm $O_2$ enthält, beispielsweise 1 bis 100, bevorzugt 1 bis 20 Vol.-ppm. Als Inertgas für die praktisch sauerstofffreie Atmosphäre können beispielsweise Stickstoff, Kohlendioxid oder Edelgase, bevorzugt Stickstoff, verwendet werden, die sauerstofffrei sind oder deren Sauerstoffgehalt in dem genannten Bereich liegt.

In dem beschriebenen Umluftofen wird zur Trocknung des getränkten Trägers eine Temperatur von etwa 50 bis etwa 120°C, bevorzugt 90 bis 120°C, besonders bevorzugt 100 bis 110°C, eingestellt. Die Dauer des Trocknungsvorganges ist abhängig von der Menge des getränkten Trägers, der Menge Wasser in der Tränkungsflüssigkeit, der Menge des Uluftstromes und der Höhe der Temperatur innerhalb des angegebenen Bereiches.

Diese Zeit kann im Bereich von etwa 1 bis etwa 4 Stunden liegen.

Danach wird der getränkte und getrocknete Katalysatorträger einer Vorzersetzung der organischen Bestandteile ebenfalls in praktisch sauerstofffreier Atmosphäre unterworfen. Hierzu wird die Temperatur zunächst in den Bereich von 140 bis 220, bevorzugt 140 bis 160°C, besonders bevorzugt 145 bis 155°C angehoben und für ½ bis 2 Stunden in diesem Bereich belassen. Danach wird mit einer Aufheizrate von 70 bis 150, bevorzugt 90 bis 110°C pro Stunde eine zweite Vorzersetzungs-Temperatur eingestellt. Diese zweite Temperatur liegt beispielsweise im Bereich von 400 bis 500, bevorzugt 430 bis 470°C und wird wie die erste Vorzersetzungs-Temperatur für ½ bis 2 Stunden eingehalten. Die Wirkungsweise der beiden beschriebenen separaten Temperaturbereiche ist noch nicht abschliessend untersucht. Es kann jedoch angenommen werden, dass im ersten der angegebenen Vorzersetzungs-Temperaturbereiche eine schonende Zersetzung des organischen Materials begonnen wird, wobei ein Zusammenwachsen von Silberkristallen zu grösseren Einheiten weitgehend vermieden wird. Im zweiten der genannten Vorzersetzungs-Temperaturbereiche kann sodann eine weitere Zersetzung des organischen Materials und eine Entfernung der entstehenden flüchtigen Brenzprodukte angenommen werden. Für den Fall, dass Promotormetalle im Katalysator vorhanden sind, kann weiterhin angenommen werden, dass im zweiten der genannten Temperaturbereiche Festkörperreaktionen zwischen den Promotormetallverbindungen und dem Silber stattfinden.

Nach dieser erfindungsgemässen Vorzersetzung enthält das dabei erhaltene Katalysator-Zwischenprodukt noch 0,5 bis 8, bevorzugt 1 bis 8, besonders bevorzugt 2 bis 5, Gew.-% Kohlenstoff, bezogen auf das Gesamtgewicht des Katalysator-Zwischenprodukts.

Im Anschluss an die beschriebene Vorzersetzung wird das Katalysator-Zwischenprodukt durch Erhitzen in einer sauerstoffhaltigen Atmosphäre aktiviert. Für dieses Erhitzen wird das Katalysator-Zwischenprodukt zunächst in der praktisch sauerstofffreien Atmosphäre auf eine Temperatur von höchstens 130°C gebracht und sodann in Gegenwart von Sauerstoff bei einer von mindestens 130°C auf maximal 300°C ständig steigenden Temperatur behandelt, wobei eine Aufheizrate von 3 bis 8°C pro Stunde eingehalten werden. Bevorzugt wird der beschriebene Temperaturanstieg von mindestens 140 bis maximal 260°C, besonders bevorzugt von mindestens 150 bis maximal 240°C vorgenommen.

Zu Beginn des beschriebenen Aufheizprogrammes wird die praktisch sauerstofffreie Atmosphäre durch eine zunächst etwa 0,4 Vol.-% Sauerstoff enthaltende Atmosphäre ersetzt, wobei der Rest zu 100 Vol.-% beispielsweise aus den oben genannten Inertgasen bestehen kann. Dieser Sauerstoffgehalt in der Aktivierungs-Atmosphäre wird sodann langsam von den genannten 0,4 bis auf 21 Vol.-% in einer solchen Weise gesteigert, dass im Abgas dieser Aktivierungsstufe ein $CO_2$-Gehalt von 1 Vol.-% nicht überschritten wird. Die Dauer dieser erfindungsgemässen Aktivierung kann aus der Aufheizgeschwindigkeit und dem gewählten Temperaturintervall abgeleitet werden und beträgt beispielsweise 12 bis 30 Stunden.

Die erfindungsgemässe Vorzersetzung und Aktivierung können unabhängig voneinander jeweils bei Normaldruck, Unter- oder Überdruck durchgeführt werden. Hierfür sei beispielsweise ein Bereich von 0,1 bis 50 bar, bevorzugt 1–20 bar, besonders bevorzugt 5–15 bar, genannt.

Im erfindungsgemässen Verfahren ist es nicht erforderlich, die Vorzersetzung und die Aktivierung zum fertigen Katalysator unmittelbar hintereinander durchzuführen. Es ist also beispielsweise möglich, nach dem Durchlaufen des zweiten Vorzersetzungs-Temperaturbereiches in praktisch sauerstofffreier Atmosphäre das hierbei erhaltene Katalysator-Zwischenprodukt unter praktisch sauerstofffreier Atmosphäre bis auf 70 bis 80°C oder tiefer abzukühlen und sodann als Zwischenprodukt aus dem Umluftofen zu nehmen. Dieses Katalysator-Zwischenprodukt enthält wie beschrieben 0,5 bis 8, bevorzugt 1 bis 8, besonders bevorzugt 2 bis 5 Gew.-% Kohlenstoff, bezogen auf das Gesamtgewicht dieses Zwischenproduktes. Dieses Zwischenprodukt ist ohne Einbusse an katalytischer Aktivität des daraus herstellbaren aktiven Silberkatalysators beliebig lagerfähig. Das Zwischenprodukt wird vor seinem Einsatz zu dem endgültigen Katalysator aktiviert. Diese Aktivierung kann beispielsweise in dem beschriebenen Umluftofen unter den beschriebenen Bedingungen erfolgen.

Die Aktivierung des Katalysator-Zwischenprodukts kann jedoch auch in dem Reaktor erfolgen, in dem der nach der Aktivierung fertige Katalysator für die vorbestimmte katalytische Reaktion eingesetzt wird, beispielsweise in einem Reaktor zur Alkylenoxid-Herstellung, sofern ein solcher Reaktor die Einhaltung der oben beschriebenen Aktivierungsbedingungen in bezug auf Temperaturführung und die Dosierung des Sauerstoffes ermöglicht. Diese zuletzt genannte Verfahrensweise zur Aktivierung des Katalysator-Zwischenprodukts ist bevorzugt.

Beispielsweise kann das Katalysator-Zwischenprodukt, gegebenenfalls nach einer längeren Zwischenlagerung, in das an sich bekannte Röhrensystem eines Festbettreaktors zur Ethylenoxid-Herstellung eingefüllt werden. Das Zwischenprodukt wird dann unter einem praktisch sauerstofffreien Inertgasstrom auf mindestens 130°C aufgewärmt. Das Aufheizen kann durch den Inertgasstrom erfolgen, jedoch auch durch ein um die Rohre zirkulierendes Wärmeträgermedium unterstützt werden. Sodann wird die bereits beschriebene Steigerung der Temperatur mit der beschriebenen Aufheizrate vorgenommen und eine Sauerstoffkonzentration von anfänglich etwa 0,4 Vol.-% eingestellt. Der Abgasstrom des Reaktors wird ständig auf seinen $CO_2$-Gehalt überprüft, der erfindungsgemäss 1 Vol.-% nicht überschreiten soll. In der beschriebenen Weise wird sodann unter Einhaltung dieses $CO_2$-Gehaltes der Sauerstoffgehalt im Reaktoreingangsgas bis auf etwa 21 Vol.-% gesteigert. Wenn nach Erreichen des oberen Temperaturwertes in der Aktivierungsstufe der $CO_2$-Gehalt des Reaktorabgases auf einen Wert von unter 0,3 Vol.-%, beispielsweise auf etwa 0,1 abgesunken ist, wird die Aktivierung des Katalysators beendet. Die Temperatur im Reaktor wird sodann auf die für die Alkylenoxid-Herstellung benötigte Temperatur, beispielsweise die für die Ethylenoxid-Herstellung benötigte Temperatur abgesenkt und die Herstellung von Alkylenoxid, beispielsweise Ethylenoxid, durch Einleiten der dem Fachmann bekannten Gasmischung zur Alkylenoxidherstellung in das Katalysatorbett begonnen.

Die Erfindung betrifft daher weiterhin Zwischenprodukte für silberhaltige und gegebenenfalls Promotoren enthaltende Trägerkatalysatoren, die durch die erfindungsgemässe und oben beschriebene Tränkung, Trocknung, Vorzersetzung und anschliessende Abkühlung in praktisch sauerstofffreier Atmosphäre auf eine Temperatur von 70 bis 80°C oder tiefer gekennzeichnet sind.

Die Erfindung betrifft ebenso ein Verfahren zur Herstellung solcher Katalysator-Zwischenpro-

dukte, das in gleicher Weise durch die erfindungsgemässe und oben beschriebene Tränkung, Trocknung, Vorzersetzung und Abkühlung unter praktisch sauerstofffreier Atmosphäre auf 70 bis 80°C oder tiefer gekennzeichnet ist.

Die erfindungsgemässen Katalysatoren zeigen im Vergleich zu den nach üblichen Herstellungsverfahren erhaltenen Katalysatoren deutliche Unterschiede in ihrer spezifischen Oberfläche, der Morphologie der Silberoberfläche und der Teilchengrösse der Silberkristallite. So liegt die spezifische Oberfläche, gemessen nach den BET-Verfahren (J. Am. Chem. Soc. 60, 309–316 [1938]) bei 0,4 bis 0,5 m²/g gegenüber einer spezifischen Oberfläche von weniger als 0,1 m²/g bei Katalysatoren, die nach üblichen Verfahren hergestellt wurden. Die mikroskopische Untersuchung zeigt bei den erfindungsgemässen Katalysatoren eine gleichmässige, kontinuierliche Teilchenstruktur mit sphärischen Silberkristalliten, die einen mittleren Durchmesser von 0,3 bis 0,4 $\mu$m aufweisen, während Katalysatoren, die nach den üblichen Verfahren erhalten worden sind, in der Morphologie der Silberoberfläche einen glasartigen Überzug mit Silberkristalliten zeigen, die einen mittleren Durchmesser von 0,7 bis 2 $\mu$m haben.

Die erfindungsgemässen Katalysatoren sind beispielsweise für die Herstellung von Alkylenoxiden durch Dampfphasenoxidation von Olefinen mit Luft bzw. mit anderen molekularen Sauerstoff enthaltenden Gasen verwendbar. Als Alkylenoxide seien beispielsweise Ethylenoxid, Propylenoxid, 1,2-Butylenoxid oder 2,3-Butylenoxid, bevorzugt Ethylenoxid, genannt. Als hierzu einsetzbare Olefine seien beispielsweise Ethylen, Propylen, 1,2-Butylen oder 2,3-Butylen, bevorzugt Ethylen, genannt. Die Erfindung betrifft somit auch die Verwendung der erfindungsgemässen Katalysatoren für die beschriebene Herstellung von Alkylenoxiden.

Diese erfindungsgemässe Verwendung ist durch die überraschend hohe Aktivität und hohe Selektivität in bezug auf die Alkylenoxid-Ausbeute besonders vorteilhaft. So ist die Arbeitstemperatur bei der Ethylenoxid-Herstellung unter Verwendung der erfindungsgemässen Katalysatoren bei 160 bis 230, bevorzugt 180 bis 220°C einstellbar, während übliche Arbeitstemperaturen bei Verfahren nach dem Stande der Technik bei 230 bis 260°C liegen. Die Selektivität beispielsweise für die Ethylenoxidherstellung liegt hierbei bei 80 bis 81%.

Infolge der tieferen Arbeitstemperatur wird bei Verwendung der erfindungsgemässen Katalysatoren die Bildung unerwünschter Nebenprodukte zurückgedrängt. Ebenso wird die Bildung von Heissstellen im Katalysatorbett zurückgedrängt, wobei gleichzeitig die Sicherheit des Alkylenoxid-Herstellungsprozesses gefördert wird.

Die erfindungsgemässe Vorzersetzung und Aktivierung des erfindungsgemässen Katalysators verläuft unter äusserst schonenden Bedingungen, die zu den beschriebenen speziellen Eigenschaften der erfindungsgemässen Katalysatoren führen. Im Gegensatz hierzu konnte bei den herkömmlichen Verfahren der unkontrollierten Zersetzung und Aktivierung von silberhaltigen Trägerkatalysatoren wegen der stark exotherm verlaufenden und durch das anwesende Silber in unkontrollierter Weise katalysierten Verbrennung von Kohlenstoff zu Kohlendioxid punktweise auftretende sehr hohe Temperaturen wegen der fehlenden Temperatur- und Sauerstoff-Kontrolle nicht vermieden werden. Solche Unregelmässigkeiten bei der Katalysator-Herstellung wirken sich jedoch sehr nachteilig auf die für die katalytische Aktivität verantwortliche Struktur der Silberkristalle aus.

In der bevorzugten Verfahrensvariante, in der unter Benutzung des erfindungsgemässen Katalysator-Zwischenproduktes die Aktivierung dieses Zwischenproduktes im Alkylenoxid-Reaktor vorgenommen wird, zeigt sich ein weiterer bedeutsamer Vorteil der vorliegenden Erfindung: Der jedem Fachmann bekannte sehr kritische Umgang mit aktivierten Katalysatoren, beispielsweise beim Lagern, beim Transport oder beim Einfüllen in den Reaktor, entfällt hierbei. Gleichzeitig sind alle beim Umgang mit aktivierten Katalysatoren möglichen irreversiblen nachteiligen Beeinflussungen ausgeschlossen, so dass erfindungsgemässe Katalysatoren nach der bevorzugten Verfahrensvariante in einem voll aktiven, durch nichts beeinträchtigten Zustand zum bestimmungsgemässen Einsatz gelangen.

Beispiel 1

Zu 190 g einer 16,7 Gew.-%igen wässrigen $AgNO_3$-Lösung werden 170 g einer 5 Gew.-%igen wässrigen NaOH-Lösung gegeben. Das gefällte Silberoxid wird nitratfrei gewaschen und in einem Separator vom Wasser getrennt. In 22 g vorgelegter 100%iger Milchsäure, die zu 80% die optisch aktive L(+)-Konfiguration aufweist, werden 321 mg Bariumhydroxid als $Ba(OH)_2 \cdot 8H_2O$ unter Rühren eingetragen. Anschliessend werden 20 mg Cäsiumcarbonat ebenfalls unter Rühren eingebracht. Der Silberoxid-Niederschlag wird in die so angesetzte Milchsäure eingetragen. Die Temperatur der Lösung wird zwischen 40° bis 70°C gehalten. Zum Schluss werden ca. 1 ml $H_2O_2$ (30%ig) unter Rühren zugesetzt. Die klare gelbgefärbte Lösung wird durch Zugabe von Wasser auf einen Gehalt von 38 Gew.-% Silber eingestellt. Mit der erhaltenen Silberlactat-Lösung werden 105 g $\alpha$-$Al_2O_3$ imprägniert. Nach Abtropfen überschüssiger Silberlactatlösung wird der getränkte Träger in einem Umluftofen unter Stickstoff bei etwa 80°C 2 Stunden getrocknet. Der Restsauerstoffgehalt bleibt hierbei unter 100 ppm.

Nach dem Trocknen wird die Temperatur innerhalb von 2 Stunden auf 220°C angehoben und dann für 2 Stunden gehalten. Danach wird mit einer Aufheizrate von 100°C/h weiterhin unter Stickstoff bis auf 450°C aufgeheizt und diese Temperatur ebenfalls 1 Stunde gehalten und anschliessend auf 70–80°C abgekühlt. Dieses Zwischenprodukt, das noch etwa 3 Gew.-% Kohlenstoff enthält, wird in den Versuchsreaktor eingefüllt. Der Reaktor wird mit Stickstoff beaufschlagt (Raum-Zeit-Geschwindigkeit: 200 bis 1000 l/h) und die Temperatur über das Heizmedium auf 150°C angehoben. Der Restsauerstoff im Stickstoff bleibt hierbei unter 100 ppm.

Mit dem Inertgas wird nun dosiert soviel Sauerstoff zugeführt, dass die Sauerstoffeingangskonzentration bei 0,4 bis 6 Vol.-% liegt. Die Bildung von $CO_2$ wird analytisch verfolgt. Der $CO_2$-Gehalt darf 1 Vol.-% nicht überschreiten. Entsprechend dem $CO_2$-Gehalt wird die Temperatur um ca. 5°C/h angehoben. Die Sauerstoff-Zudosierung wird ebenfalls um ca. 1 Vol.-%/h erhöht. Nach Erreichung einer Endtemperatur von 240°C und eines Sauerstoffgehaltes von 21 Vol.-% wird 4 bis 6 Stunden, nachdem der $CO_2$-Gehalt unter 0,1 Vol.-% abgesunken ist, die Aktivierung des Zwischenproduktes beendet. Die Temperatur im Reaktor wird auf 160°C abgesenkt und das für die Umsetzung zu Ethylenoxid erforderliche Gasgemisch wird über den Katalysator geleitet. Nach einer Konditionierungszeit von ca. 48 Stunden erreicht der so hergestellte Katalysator seine endgültige Aktivität und Selektivität. Der so hergestellte Katalysator erhält die Bezeichnung A. Die Versuchsergebnisse stehen in Tabelle 1.

### Beispiel 2

Es wird ein Katalysator hergestellt wie in Beispiel 1. Der Milchsäure wird neben Barium und Cäsium noch 8 g Saccharose zugesetzt. Der Katalysator erhält die Bezeichnung B.

### Beispiel 3 (zum Vergleich)

Es wird ein getränkter Katalysatorträger hergestellt wie in Beispiel 1. Die Zersetzung des imprägnierten Trägers erfolgt in einem Umluftofen in Gegenwart von Luft. Mit einer Aufheizrate von 100°C/h wird bis auf 400°C aufgeheizt und 1 Stunde bei dieser Temperatur belassen und anschliessend abgekühlt. Der Katalysator erhält die Bezeichnung C.

### Beispiel 4 (Anwendungsbeispiel)

Der Laborversuchsreaktor besteht aus einem ölbeheizten Glasrohr mit Doppelmantel mit einer lichten Weite von 50 mm und einer Länge von 1000 mm, von der 100 mm mit einem inerten Trägermaterial gefüllt sind und als Vorheizzone dienen. Die Laborversuche wurden bei atmosphärischem Druck durchgeführt. Die analytische Überwachung des Reaktorproduktgasstromes erfolgt kontinuierlich durch einen Prozessgaschromatographen. Die Raum-Zeit-Geschwindigkeit beträgt: 250 Raumteile Gas pro Raumteil Katalysator und Stunde. Das eingesetzte Gasgemisch für die Gasphasenoxidation am Katalysator besteht aus:

| | |
|---|---|
| $C_2H_4$ | 30 Vol.-% |
| $O_2$ | 8 Vol.-% |
| $CH_4$ | 50 Vol.-% |
| $N_2$ + Inerte | 12 Vol.-% |

Dem eingesetzten Gasgemisch wurde als Inhibitor 1 bis 2 ppm 1,2-Dichlorethan zugesetzt.

Tabelle 1

| Katalysator | Temperatur (°C) | EOX (Vol.-%)* | Selektivität |
|---|---|---|---|
| A | 160 | 1,2 | 81 |
| A | 180 | 1,7 | 80,5 |
| A | 200 | 2,0 | 80,0 |
| A | 205 | 2,08 | 80,0 |
| B | 160 | 1,5 | 81,5 |
| B | 180 | 2,0 | 80,9 |
| B | 190 | 2,3 | 80,4 |
| B | 200 | 2,5 | 80,6 |
| C | 240 | 2,2 | 79,0 |

Die Katalysatoren zeigten nach 3monatigem Betrieb bei den verschiedenen angegebenen Temperaturen keinen Aktivitätsverlust.

* EOX = Ethylenoxidgehalt des den Reaktor verlassenden Gasstroms

### Patentansprüche

1. Silberhaltige und gegebenenfalls Promotoren enthaltende Trägerkatalysatoren, hergestellt durch

a) Tränken eines Trägers mit einer spezifischen Oberfläche von höchstens 1 m²/g mit einer Silber- und gegebenenfalls Promotormetallionen enthaltenden Milchsäure,

b) Trocknen des nach a) erhaltenen getränkten Trägers und Vorzersetzen der in ionischer und freier Form vorliegenden Milchsäure in praktisch sauerstofffreier Atmosphäre, wobei das Trocknen bei einer Temperatur von 50 bis 120°C vorgenommen wird und die Vorzersetzung nacheinander in den beiden Temperaturbereichen von 140 bis 220°C und 400 bis 500°C durchgeführt wird, wobei man für den Übergang zwischen beiden Temperaturbereichen eine Aufheizrate von 70 bis 150°C/h einstellt, und

c) Aktivierung der nach b) erhaltenen Katalysator-Zwischenprodukte durch Erhitzen in sauerstoffhaltiger Atmosphäre, wobei die Temperatur von mindestens 130°C auf maximal 300°C mit einer Aufheizrate von 3 bis 8°C/h gesteigert wird und der Sauerstoffgehalt von 0,4 bis 21 Vol.-% in einer solchen Weise gesteigert wird, dass im Abgas der Aktivierungsstufe ein $CO_2$-Gehalt von 1 Vol.-% nicht überschritten wird.

2. Katalysatoren nach Anspruch 1, dadurch gekennzeichnet, dass eine Barium und Cäsium als Promotoren enthaltende Milchsäure eingesetzt wird.

3. Zwischenprodukte für silberhaltige und gegebenenfalls Promotoren enthaltende Trägerkatalysatoren, hergestellt durch

a) Tränken eines Trägers mit einer spezifischen Oberfläche von höchstens 1 cm²/g mit einer Silber- und gegebenenfalls Promotormetallionen enthaltenden Milchsäure,

b) Trocknen des nach a) erhaltenen getränkten Trägers und Vorzersetzen der in ionischer und freier Form vorliegenden Milchsäure in praktisch sauerstofffreier Atmosphäre, wobei das Trocknen bei einer Temperatur von 50 bis 120°C vorgenommen wird und die Vorzersetzung nacheinander in den beiden Temperaturbereichen von 140 bis 220°C und 400 bis 500°C durchgeführt wird, wobei man für den Übergang zwischen beiden Temperaturbereichen eine Aufheizrate von 70 bis 150°C/h einstellt, und anschliessendes Abkühlen in praktisch sauerstofffreier Atmosphäre auf 70 bis 80°C oder darunter.

4. Verfahren zur Herstellung von silberhaltigen und gegebenenfalls Promotoren enthaltenden Trägerkatalysatoren durch Tränken eines Trägers mit einer spezifischen Oberfläche von höchstens 1 m²/g mit einer Silber und gegebenenfalls Promotormetalle enthaltenden Milchsäure-Lösung, anschliessende Trocknung und Kalzinierung, dadurch gekennzeichnet, dass man

a) den getränkten Träger in einer praktisch sauerstofffreien Atmosphäre bei 50 bis 120°C trocknet und die in ionischer oder freier Form vorliegende Milchsäure nacheinander in den beiden Temperaturbereichen von 140 bis 220°C und 400 bis 500°C in praktisch sauerstofffreier Atmosphäre vorzersetzt, wobei man für den Übergang zwischen den beiden Temperaturbereichen eine Aufheizrate von 70 bis 150°C/h einstellt und

b) das nach a) erhaltene Katalysator-Zwischenprodukt durch Erhitzen in einer sauerstoffhaltigen Atmosphäre aktiviert, wobei die Temperatur von mindestens 130°C auf maximal 300°C mit einer Aufheizrate von 3 bis 8°C/h gesteigert wird und der Sauerstoffgehalt von 0,4 bis 21 Vol.-% in einer solchen Weise gesteigert wird, dass im Abgas der Aktivierungsstufe ein $CO_2$-Gehalt von 1 Vol.-% nicht überschritten wird.

5. Verfahren nach Anspruch 4, dadurch gekennzeichnet, dass eine Milchsäure mit einem Gehalt an optisch aktiver Milchsäure eingesetzt wird.

6. Verfahren nach Anspruch 4 und 5, dadurch gekennzeichnet, dass in der eingesetzten Milchsäure mindestens 50% aus der L(+)-Form besteht, während der Rest das Racemat darstellt.

7. Verfahren nach Anspruch 4 bis 6, dadurch gekennzeichnet, dass die Aktivierung des Katalysator-Zwischenproduktes, gegebenenfalls nach einer Zwischenlagerung des Zwischenprodukts, in einem Reaktor für die Alkylenoxid-Herstellung unter Druck bevorzugt bei 10–15 bar vorgenommen wird.

8. Verfahren nach Anspruch 4 bis 6, dadurch gekennzeichnet, dass der das Silber und gegebenenfalls die Promotormetallionen enthaltenden Milchsäure vor dem Tränken in Milchsäure lösliche, leicht zersetzliche und nicht reduzierende organische Verbindungen zugesetzt werden.

9. Verfahren zur Herstellung eines silberhaltigen und gegebenenfalls Promotormetallverbindungen enthaltenden Katalysator-Zwischenprodukts durch Tränken eines Trägers mit einer spezifischen Oberfläche von höchstens 1 m²/g mit einer Silber und gegebenenfalls Promotormetalle enthaltenden Milchsäure-Lösung und anschliessende thermische Behandlung, dadurch gekennzeichnet, dass man den getränkten Träger in einer praktisch sauerstofffreien Atmosphäre bei 50 bis 120°C trocknet und die in ionischer oder freier Form vorliegende Milchsäure nacheinander in den beiden Temperaturbereichen von 140 bis 220°C und 400 bis 500°C in praktisch sauerstofffreier Atmosphäre vorzersetzt, wobei man für den Übergang zwischen beiden Temperaturbereichen eine Aufheizrate von 70 bis 150°C/h einstellt, und anschliessend in praktisch sauerstofffreier Atmosphäre auf 70 bis 80°C oder tiefer abkühlt.

10. Verwendung der Katalysatoren nach Anspruch 1 zur Herstellung von Alkylenoxiden durch Oxidation von Alkenen mit Sauerstoff enthaltenden Gasen.

**Revendications**

1. Catalyseurs sur supports contenant de l'argent et contenant éventuellement des promoteurs, catalyseurs préparés par:

a) l'imprégnation d'un support, ayant une surface spécifique de contact d'au maximum 1 m²/g, à l'aide d'un acide lactique contenant des ions argent et éventuellement des ions de métaux promoteurs,

b) séchage du support imprégné, obtenu selon (a) et décomposition préliminaire de l'acide lactique, présent sous forme ionique et sous forme libre, dans une atmosphère pratiquement sans oxygène, le séchage étant effectué à une température de 50° à 120°C et la décomposition préliminaire successivement dans les deux gammes de température comprises entre 140 et 220°C, d'une part, et 400 et 500°C, d'autre part, avec, pour le passage entre les deux gammes de température, établissement d'une vitesse de chauffage de 70 à 150°C/h, et

c) activation des produits intermédiaires de catalyseur, obtenus selon (b), par chauffage en atmosphère contenant de l'oxygène, la température étant élevée d'une valeur minimale de 130°C à une valeur maximale de 300°C à une vitesse de chauffage de 3 à 8°C par heure, et la teneur en oxygène étant élevée de 0,4 à 21% en volume de manière à ne pas excéder dans le gaz sortant de l'étape d'activation une teneur en $CO_2$ de 1% en volume.

2. Catalyseurs selon la revendication 1, caractérisés en ce qu'on utilise un acide lactique contenant du baryum et du césium comme promoteurs.

3. Produits intermédiaires pour l'obtention de catalyseurs sur supports contenant de l'argent et contenant éventuellement des promoteurs, produits préparés par:

a) imprégnation d'un support, présentant une surface spécifique de contact d'au maximum 1 cm²/g, à l'aide d'un acide lactique contenant de l'argent et contenant éventuellement des ions métalliques promoteurs,

b) séchage du support imprégné obtenu (a) et décomposition préliminaire de l'acide lactique, présent sous forme ionique et sous forme libre dans une atmosphère pratiquement sans oxygène, le séchage étant effectué à une température de 50 à 120°C et la décomposition préliminaire successivement dans deux gammes de température allant de 140 à 220°C, d'une part, et de 400 à 500°C, d'autre part, en établissant pour le passage entre les deux gammes de température une vitesse de chauffage de 70 à 150°C/h, puis refroidissement en atmosphère pratiquement sans oxygène jusqu'à 70 à 80°C ou jusqu'à une température plus basse.

4. Procédé de production de catalyseurs sur supports, contenant de l'argent et contenant éventuellement des promoteurs, par imprégnation d'un support, présentant une surface spécifique de contact d'au maximum 1 m²/g, à l'aide d'une solution d'acide lactique contenant de l'argent et contenant éventuellement des métaux comme promoteurs, puis séchage et calcination, procédé caractérisé en ce que:

a) on sèche entre 50 et 120°C, dans une atmosphère pratiquement sans oxygène, le support imprégné et l'on soumet à une décomposition préliminaire l'acide lactique, présent sous forme ionique ou sous forme libre, on opérant succéssivement dans les deux gammes de températures comprises entre 140 et 220°C, d'une part, et entre 400 et 500°C, d'autre part, en atmosphère pratiquement sans oxygène, en établissant pour le passage entre les deux gammes de température une vitesse de chauffage de 70 à 150°C/h, et

b) on active le produit intermédiaire de catalyseur, obtenu selon (a), en le chauffant dans une atmosphère contenant de l'oxygène et en élevant, à une vitesse de chauffage de 3 à 8° par heure, la température de 130°C au minimum à 300°C au maximum et en élevant la teneur on oxygène de 0,4 à 21% en volume de manière à ne pas excéder, dans le gaz sortant de l'étape d'activation, une teneur en $CO_2$ de 1% en volume.

5. Procédé selon la revendication 4, caractérisé en ce qu'on utilise un acide lactique contenant un acide lactique optiquement actif.

6. Procédé selon l'une des revendications 4 et 5, caractérisé en ce que, dans l'acide lactique que l'on utilise, au moins 50% consistent en la forme L(+), cependant que le reste représente le racémique.

7. Procédé selon l'une quelconque des revendications 4 à 6, caractérisé en ce qu'on effectue l'activation du produit intermédiaire de catalyseur, éventuellement après un stockage intermédiaire de ce produit intermédiaire, dans un réacteur de production d'un oxyde d'alkylène, sous une pression qui est de préférence de 10 à 15 bars.

8. Procédé selon l'une quelconque des revendications 4 à 6, caractérisé en ce qu'on ajoute à l'acide lactique, contenant l'argent et éventuellement des ions de métaux promoteurs, avant l'opération d'imprégnation dans l'acide lactique, des composés organiques solubles, pouvant facilement se décomposer et qui ne se réduisent pas.

9. Procédé pour préparer un produit intermédiaire de catalyseur, contenant de l'argent et contenant éventuellement des composés de métaux promoteurs, par imprégnation d'un support, présentant une surface spécifique de contact d'au maximum 1 m²/g, à l'aide d'une solution d'acide lactique contenant de l'argent et éventuellement des métaux promoteurs puis traitment thermique, procédé caractérisé en ce qu'on sèche dans une atmosphère pratiquement sans oxygène, entre 50 et 120°C, le support imprégné et l'on soumet à décomposition préliminaire, dans une atmosphère pratiquement sans oxygène, successivement dans les deux gammes de température comprises d'une part entre 140 et 220°C et d'autre part entre 400 et 500°C, l'acide lactique présent sous forme ionique ou sous forme libre, en établissant, pour le passage entre les deux gammes de températures, une vitesse de chauffage de 70 à 150°C/h, et l'on refroidit ensuite, dans une atmosphère pratiquement sans oxygène, jusqu'à 70 à 80°C ou à une température plus faible.

10. Utilisation des catalyseurs selon la revendication 1 pour produire des oxydes d'alkylènes par oxydation d'alcènes à l'aide de gaz contenant de l'oxygène.

**Claims**

1. Supported catalysts containing silver and, if appropriate, promoters, prepared by

a) impregnating a support having a specific surface area of at most 1m²/g with a lactic acid containing silver and, if appropriate, promoter metal ions,

b) drying the impregnated support obtained according to a) and preliminarily decomposing the lactic acid, present in ionic form and in the free form, in a virtually oxygen-free atmosphere, the drying being carried out at a temperature of 50 to 120°C and the preliminary decomposition being carried out successively in the two temperature ranges of 140 to 220°C and 400 to 500°C, a heating up rate of 70 to 150°C/hour being established for the transition between the two temperature ranges, and

c) activating the catalyst intermediate products obtained according to b) by heating in an oxygen-containing atmosphere, the temperature being increased from at least 130°C to at most 300°C at a heating up rate of 3 to 8°C/hour and the oxygen content being increased from 0.4 to 21% by volume in a manner such that the $CO_2$ content of the off-gas from the activation stage does not exceed 1% by volume.

2. Catalysts according to Claim 1, characterised in that a lactic acid containing barium and cesium as promoters is employed.

3. Intermediate products for supported catalysts containing silver and, if appropriate, promoters, prepared by

a) impregnating a support having a specific surface area of at most 1 cm²/g with a lactic acid containing silver and, if appropriate, promoter metal ions,

b) drying the impregnated support obtained according to a) and preliminarily decomposing the lactic acid, present in ionic form and in the free from, in a virtually oxygen-free atmosphere, the drying being carried out at a temperature of 50 to 120°C and the preliminary decomposition being carried out successively in the two temperature ranges of 140 to 220°C and 400 to 500°C, a heating up rate of 70 to 150°C/hour being established for the transition between the two temperature ranges, and then cooling the intermediate product in a virtually oxygen-free atmosphere to 70 to 80°C or below.

4. Process for the preparation of supported catalysts containing silver and, if appropriate, promoters by impregnating a support having a specific surface area of at most 1 m²/g with a lactic acid solution containing silver and, if appropriate, promoter metals and then drying and calcining the support, characterised in that

a) the impregnated support is dried in a virtually oxygen-free atmosphere at 50 to 120°C and the lactic acid, present in ionic form or in the free form, is preliminarily decomposed successively in the two temperature ranges of 140 to 200°C and 400 to 500°C in a virtually oxygen-free atmosphere, a heating up rate of 70 to 150°C/hour being established for the transition between the two temperature ranges and

b) the catalyst intermediate product obtained according to a) is activated by being heated in an oxygen-containing atmosphere, the temperature being increased from at least 130°C to at most 300°C at a heating up rate of 3 to 8°/hour and the oxygen content being increased from 0.4 to 21% by volume in a manner such that the $CO_2$ content

of the off-gas from the activation stage does not exceed 1% by volume.

5. Process according to Claim 4, characterised in that a lactic acid containing optically active lactic acid is employed.

6. Process according to Claim 4 and 5, characterised in that at least 50% of the lactic acid employed consists of the L(+)-form, whilst the remainder is the racemate.

7. Process according to Claim 4 to 6, characterised in that the activation of the catalyst intermediate product, if appropriate after intermediate storage of the intermediate product, is carried out under pressure, preferably under 10–15 bars, in a reactor for the preparation of alkylene oxides.

8. Process according to Claims 4 to 6, characterised in that soluble, readily decomposable and non-reducing organic compounds are added to the lactic acid containing the silver, and, if appropriate, the promoter metal ions, before the support is impregnated in the lactic acid.

9. Process for the preparation of a catalyst intermediate product containing silver and, if appropriate, promoter metal compounds by impregnating a support having a specific surface area of at most 1 m²/g with a lactic acid solution containing silver and, if appropriate, promoter metals and then subjecting the support to heat treatment, characterised in that the impregnated support is dried in a virtually oxygen-free atmosphere at 50 to 120°C and the lactic acid, present in ionic form or in the free form, is preliminarily decomposed, successively in the two temperature ranges of 140 to 220°C and 400 to 500°C, in a practically oxygen-free atmosphere, a heating up rate of 70 to 150°C/hour being established for the transition between the two temperature ranges, and the support is then cooled in a virtually oxygen-free atmosphere to 70 to 80°C or below.

10. Use of the catalysts according to Claim 1 for the preparation of alkylene oxides by oxidation of alkenes with oxygen-containing gases.